# NEW EUROPEAN PATENT SPECIFICATION

(11) **EP 1 796 607 B2**
(45) Date of publication and mention of the opposition decision: **30.04.2014**
(45) Mention of the grant of the patent: 22.07.2009
(21) Application number: 05794702.0
(22) Date of filing: 26.09.2005
(51) Int. Cl.: A61F 5/445

(54) **A resealable ostomy appliance**
Wiederverschließbare Ostomievorrichtung
Dispositif de stomie refermable

(30) Priority: 29.09.2004 DK 200401485
(43) Date of publication of application: 20.06.2007
(73) Proprietor: Coloplast A/S, 3050 Humlebaek (DK)
(72) Inventor: CIOK, Danuta, DK-2990 Nivaa (DK); STROEBECH, Esben, DK-2970 Hoersholm (DK)
(86) International application number: PCT/EP2005/054815
(87) International publication number: WO 2006/035014

(56) References cited:
- EP-A- 0 259 184
- WO-A-01/54632
- WO-A2-2004/084777
- WO-A2-2005/025466
- US-A- 4 826 495
- US-A- 5 429 626
- US-A- 5 562 107
- US-A- 5 591 144

## Description

### BACKGROUND OF THE INVENTION

The present invention relates to an ostomy appliance and to a method of applying an ostomy appliance body side member around a stoma.

In connection with surgery for a number of diseases in the gastro-intestinal or urinary tract a consequence is, in many cases, that the colon, the ileum or the ureter has been exposed surgically and the patient is left with an abdominal stoma, or, in nephrostomy or ureterostomy, the ureter or a catheter is exposed in the back or the chest region or abdominal region, and the effluents or waste products of the body, which are conveyed through these organs, are discharged through the artificial orifice or opening and are collected in a collection bag, which is usually adhered to the skin by means of an adhesive wafer or plate having an inlet opening for accommodating the stoma/ureter/catheter. Also in connection with a fistula, the patient will have to rely on an appliance to collect the bodily material emerging from such opening.

Ostomy appliances are well known. Such appliances may be two-piece or one-piece appliances. In both types of appliances, an adhesive barrier member (or base plate) is attached to the wearer's abdomen/back/chest. In case of a one-piece appliance, a receiving member or bag is permanently attached to the base plate. In case of a two-piece appliance, the adhesive barrier member forms part of a body side member and a receiving member or bag is attached releasably to the body side ostomy member for receiving exudates from the stoma.

When using one-piece appliances, the whole appliance, including the adhesive skin barrier securing the appliance to the skin is normally removed and replaced by a fresh appliance. When using two-piece appliances, the body side ostomy member is left in place up to several days, and only the receiving member or bag attached to the body side member is replaced. The attachment means for attaching an ostomy receiving bag may e.g. be a system known per se comprising matching coupling rings or matching flanges and adhesive surfaces engaging with and sealing against a flange area of the body side member.

It is necessary to change the body side member of a two-piece appliance when the centre part of the adhesive wafer has been sufficiently deteriorated to allow access of the aggressive exudates to the skin surrounding the stoma, irrespective of the fact that the wafer as such has a much longer wearing time. The access of aggressive exudates to the skin is causing skin problems.

Frequent changing of the body side member of a two-piece appliance is undesirable due to the irritation of the skin and the quality of life of the user may be improved and the nuisance of the wearing of an ostomy appliance reduced if the intervals between exchanging of body side member can be increased.

The service time of the body side ostomy member depends *inter alia* of the amount and the aggressiveness of the exudates and of the sealing between the stoma and the body side ostomy member.

The sealing depends on the fit to the stoma. Conventionally, only a limited number of standard appliances having holes of different size are available and the user or an assistant must customise the body side member by cutting the edge of the hole to adapt the body side member to the stoma.

When cutting the edge of the hole of an adhesive wafer of a conventional one-piece ostomy appliance for adapting the same to the size and shape of a stoma the cutting is complicated by the fact that in order to secure discretion, for decorative purposes and for providing softness, low noise generation and comfort the bag is often made from an opaque material or covered and/or provided with a cover or front layer rendering it very difficult, if not impossible for the user or the nurse to observe the stoma area during determination of a cutting line, for adaptation of the hole, or when applying the appliance.

Published International Patent Applications Nos. WO 98/17212 and WO 98/53771 disclose ostomy appliances comprising a body side member comprising an adhesive wafer for securing the appliance to the user's skin, said wafer having a hole for receiving a stoma, and an optionally separately exchangeable receiving member or bag secured to the body side ostomy member for receiving secretions from the ostomy said ostomy appliance further comprising a sealing member disposed in the hole of the wafer surrounding the stoma and having a hole for accommodating the stoma.

The ostomy appliance disclosed in Published International Patent Application No. WO 98/53771 has a sealing member having balanced plastic and elastic properties allowing a better adaptation of the hole of the ostomy appliance to a stoma by a temporary enlarging of the hole by everting or rolling up the inner rim of the hole for accommodating the stoma.

WO 98/17212 and WO 98/53771 teach the use of a such a sealing member together with two-piece ostomy appliances.

EP-A-0259184 describes a coupling means having a retainer plate and a flexible sealing adhesive member for an ostomy appliante*.* The preamble of claim 1 is based on the disclosure of said document.

It has been found desirable in practice to make use of separate sealing members. Such separation facilitates the adaptation of an ostomy appliance, and secures the sealing between the ostomy appliance and the stoma. If used with a one-piece ostomy appliance, such a sealing member would be difficult to manipulate for several reasons. Firstly, it is very difficult to adapt the sealing member to the stoma after applying the ostomy appliance if the sealing member has a tacky surface on the side facing the bag as it will then adhere to the bag during attempts to adapt the sealing member to the stoma which must be performed by pressing the wall of the bag against the sealing member. Then, also a risk exists that the wall of the bag is tom when trying to disengage the contact. Secondly, due to the measures to secure discretion, for decorative purposes and for providing softness, low noise generation and comfort stated above it also in this case very difficult, if not impossible for the user or the nurse to observe the stoma area during adaptation of such a sealing member when applying one-piece appliances.

A known major problem with such receiving bags is that it can be difficult to dispose of the used bag in a convenient and hygienic manner. Some ostomists will cut the used bags open, e.g. by cutting off an edge thereof and depositing the contents into a WC for flushing away and disposing or depositing the empty bag in a waste bin. Such disposal of used bags and the contents therein is indeed unhygienic and unpleasant for the user, and in view of this a number of proposals have been made for ostomy bags which may be flushed down a WC.

### SUMMARY OF THE INVENTION

The present invention relates to an ostomy appliance comprising a front wall and a rear wall of flexible material forming a bag, said rear wall having an opening for receiving a stoma, and being provided with an adhesive wafer for securing the appliance to a user's skin, said wafer having a hole being aligned with the opening for receiving the stoma, wherein the opening of the rear wall has an edge being adapted to be secured to a surface of the adhesive wafer facing away from the user in an attachment zone surrounding the hole of the wafer, and wherein a first part of the edge of the opening of the rear wall is permanently secured to the surface of the wafer in a corresponding first part of the attachment zone, and a second, remaining part of the edge of the opening of the bag is prepared for adhesive reversible coupling of the same to the remaining second part of the attachment zone wherein the ostomy appliance further comprises an inner bag.

### Brief Description of the Drawings

The invention is disclosed more in detail with reference to the drawings in which Fig. 1 shows an ostomy appliance of the invention in its open state, Fig. 2 shows another embodiment of an ostomy appliance of the invention in its open state applied to a users abdomen, Fig. 3 shows further embodiment of an ostomy appliance of the invention in its dosed state, and Fig. 4 shows a stiffening element for use in an ostomy appliance of the invention.

### Detailed Description of the Present Invention

The present invention relates to an ostomy appliance comprising a front wall and a rear wall of flexible material forming a bag, said rear wall having an opening for receiving a stoma, and being provided with an adhesive wafer for securing the appliance to a user's skin, said wafer having a hole being aligned with the opening for receiving the stoma, wherein the opening of the rear wall has an edge being adapted to be secured to a surface of the adhesive wafer facing away from the user in an attachment zone surrounding the hole of the wafer, and wherein a first part of the edge of the opening of the rear wall is permanently secured to the surface of the wafer in a corresponding first part of the attachment zone, and a second, remaining part of the edge of the opening of the bag is prepared for adhesive reversible coupling of the same to the remaining second part of the attachment zone wherein the ostomy appliance further comprises an inner bag.

As a part of the edge of the bag of a one-piece appliance is not secured to the adhesive wafer, that part of the edge may be folded back giving access to the stoma area from the distal side of the appliance facing away from the user during the application of the appliance. Thus, a simple evaluation of the stoma area for cutting the wafer for adapting the same to the specific stoma and manipulation and location of an adhesive wafer in relation to the stoma is enabled. All such adaptation and application may be carried out having full view over the stoma area from the distal side. Then, the edge of the bag is easily sealed to the wafer along the remaining part of the attachment zone, and thus along the full periphery of the bag, using an adhesive. The adhesive is preferably protected by a release liner until the final sealing of the bag. In one aspect, the bag is sealed permanently to the wafer. Thus, in use, after application the product works like a conventional one-piece appliance, and advantages of access to the stoma area when working with two-piece appliances are transferred to the one-piece technology.

In another embodiment, the bag is sealed to the wafer in a way allowing the seal to be broken and re-applied. This allows the bag to be reopened for inspection of the stoma. An example of an adhesive that will allow such reopening is acrylic adhesive. Such adhesives are well known in the art.

The opening of the rear wall has an edge which is adapted to be secured to a surface of the adhesive wafer. Such securing may be achieved by attachment of the edge e.g. by means of an adhesive. In some embodiments only the edge of the rear wall is adapted to be secured to the surface of the adhesive wafer while in other embodiments also an area adjacent to the edge may be secured or attached to the surface of the adhesive wafer.

A release liner may be provided on the second remaining part of the edge of the opening of the rear wall. In order to adhere the second remaining part of the rear wall to the wafer the release liner must be removed.

In one embodiment the second, remaining part of the edge of the opening of the rear wall is adapted to be changed from a first position wherein it is not adhesively sealed to the remaining second part of the attachment zone, and a second position wherein it is adhesively sealed to the remaining second part of the attachment zone. In a special embodiment, this remaining part of the edge of the opening of the rear wall can be switched between these two positions.

Also described is that the second, remaining part of the edge of the opening of the bag is prepared for permanently adhesive sealing of the same to the remaining second part of the attachment zone. Thus in the latter embodiment when the second remaining part has been permanently adhered to the second part of the attachment zone it may be difficult or even impossible to remove it again. Accordingly if one succeeds in removing the second part it can not be re-attached again.

The resulting hole may however show an exposed edge which does not provide a sufficient sealing against a stoma ensuring that no leak occurs and may injure the surface of the stoma e.g. when the user bends or turns due to the exposed edge of the carrier sheet which after cutting even may show a serrated edge.

In one embodiment the ratio between the second remaining edge is 50% of the entire edge, that is the sum of the first edge and the second remaining edge. In another embodiment this ratio is selected from the group consisting of 40%, 41%, 42%, 43%, 44%, 45%, 46%, 47%, 48%, 49%, 51%, 52%, 53%, 54%, 55%, 56%, 57%, 58%, 59%, 60% and 65%.

In one embodiment the ostomy appliance is in the form of a set which comprises a separate sealing member for disposing in the hole of the wafer surrounding the stoma and having a hole for accommodating the stoma. The present invention enables the use of such a sealing member together with one-piece ostomy appliances and enables an easy manipulation and shaping of such sealing member before application and to seal between the adhesive wafer and the stoma avoiding direct contact between the stoma and the edge of the hole. Thus, the edge of the hole of the sealing member may be everted or rolled and the sealing member positioned from the distal side of the appliance or from "inside the bag".

The use of a separate sealing member also enables a reduction in the number of available sizes of holes as one or a few large sizes of holes may be tailored to most stomas using the separate sealing member, which has not been possible for one-piece ostomy appliances until now. Furthermore, the cutting for enlarging the hole of a one-piece ostomy appliance for accommodating a stoma is rendered easier as the fit is not critical.

The ostomy bag described above is used as an outer-bag for a two-bag system, also referred to as a flush system. The essentials thereof being that inside the ostomy bag described is placed an inner-bag. The inner-bag is preferably a disposable inner-bag having a hole for receiving wastes exiting the stoma, ureter or catheter, such hole attached to the adhesive wafer, such that waste is directed into to the inner-bag. In use, the user or nurse will apply the ostomy bag (the outer bag) as described. Then, before closing rear wall of the outer-bag, an inner-bag is placed inside the outer-bag, and the hole in the inner-bag attached to the adhesive wafer. When the inner-bag is in place, the rear wall of the outer-bag is closed.

Now, for replacing the inner-bag, the rear wall of the outer-bag is reopened, the hole in the inner-bag attached to the adhesive wafer is detached the adhesive wafer, and preferably closed/sealed, and withdrawn from the outer-bag. A new inner-bag can now be placed in the clean outer-bag as described above.

This system has numerous advantages. First, let it be recalled that the typical ostomist is elderly. Here, he is here offered the possibility to open the outer-bag while the bags are still sitting on his skin, and detach the hole of the inner-bag from the adhesive wafer, while the outer-bag is still hanging in on the skin. When ready, he can lift the inner-bag out of the outer-bag, and let it go to the toilet. Furthermore, when the outer-bag is opened by detaching at the edge from the adhesive wafer, gravity will pull the rear wall down and maintain an opening, providing an easy access to the contents of the bag.

In one embodiment, the inner-bag is biodegradable.

In accordance with a suitable embodiment of the invention, the second part of the edge of the bag is secured to one surface of a stiffening element reducing the risk of wrinkling of the edge of the bag.

When the first and the second part of the edge of the bag are secured to a first and a second part, respectively, of one surface of the stiffening element, the handling of the bag and the securing of the first part of the edge of the bag to the first part of the attachment zone are facilitated and the process is readily adapted for automatic production.

A stiffening element is preferably in the form of a ring being secured to the circumference of the opening of the bag. Such stiffening element may suitably be made from a plastics material, which is flexible and relatively rigid so that it is substantially dimensionally stable under normally occurring loads. Polyolefins, particularly polyethylene, e.g. in the form of a blend of high and low density polyethylenes, have been found suitable, but other materials having similar properties may be used. The stiffening element may alternatively be in the form of a flange of a foam material such as a cellular plastic material conventionally used for production of flanges for ostomy appliances. A polyurethane foam is suitably used.

The adhesive for adhesive sealing of the second part of the edge of the bag or the stiffening element to the second part of the attachment zone is suitably an "aggressive" adhesive material providing an immediate grip when contacted with the other surface after removing a release liner. It is preferred that the adhesive material has a sufficient thickness and is sufficiently mouldable to provide a reliable sealing of the edge of the bag. A suitable adhesive material is pressure sensitive adhesive suitable for use with the parts to be united such as acrylic adhesives, elastomeric adhesives, polyurethane adhesives, or polyester adhesives. The choice of adhesive may be made in accordance with the above requirements, as it is not critical for the invention in the respect that the adhesive is not in contact with the skin. It is also contemplated that the adhesive properties of the adhesive and the second part of the edge of the bag or the stiffening element and the second part of the attachment zone are balanced enabling an opening and resealing of the bag, if desired.

It is suitable when the stiffening element is a flat ring provided with two notches dividing the flat ring into two parts. The shape of such ring should correspond to the shape of the attachment zone and may e.g. be circular or elliptical. Such notches provide predetermined zones in which the stiffening element is readily folded back giving access to the distal side of the adhesive wafer. The notches may be present on the proximal surface of the stiffening element of on the distal surface of the stiffening element whichever deemed most appropriate. When placed on the distal surface of the stiffening element, care must be taken when securing the edge of the bag to the stiffening element that the edge of the bag is reliably sealed to the surface without canals in the notch-area. The securing may e.g. be effected by welding or suitably applying an adhesive, preferably in an amount ensuring a reliable sealing in the notch-area. A combination of a welding seam and adhesive sealing may be used if deemed appropriate.

It is especially suitable to locate the notches stretch essentially along a chord of the circular ring facilitating the bending of the stiffening element. The notches stretch essentially along a diameter of the circular ring giving a large opening to the stoma site in the ostomy appliance.

In one embodiment of the invention the part of the stiffening element secured the second part of the bag is provided with an adhesive layer being covered by a protecting layer. In case that the notches are located on the proximal surface of the stiffening element, care must be taken to ensure that the notch is sealed reliably when adhering the second of the edge of the bag to the second part of the attachment zone, e.g. by applying a sufficient amount of adhesive material to fill the notch.

A stiffening element to be used in accordance with the present invention may be made by a method known per se for manufacturing products from the selected materials such as injection moulding, die casting, pressing, or foaming using corresponding moulds. Alternatively, a stiffening element may be cut from a workpiece of the desired material such as a foam block. A notch may be prepared when forming the stiffening element or later using methods known per se such as cutting or milling.

In another embodiment of the invention the second part of the attachment zone of the wafer is provided with an adhesive layer being covered by a protecting layer. In case that the notches are located on the proximal surface of the stiffening element, care must also be taken in this case to ensure that the notch is sealed reliably when adhering the second of the edge of the bag to the second part of the attachment zone, e.g. by applying a sufficient amount of adhesive material at the site of the attachment zone near the notch to fill the notch.

In a further embodiment of the invention, the front wall is provided with an opening provided with cover, which may be released and resealed and thus giving temporary access to the internal space of the bag for inspection of the stoma area during the service time of the ostomy appliance. Such opening is especially of interest in the first period after a stoma having been made in order to allow an inspection of the newly formed stoma without having to remove the appliance and also for establishing a suitable regime for exchange of appliances for the ostomate in question. The opening may be provided with a closure corresponding to an adhesive coupling normally used for coupling of collection bags to ostomy body side members of two-piece appliances or a conventional coupling ring for coupling of collection bags to ostomy body side members of two-piece appliances and a matching closure such as the closure disclosed in WO 98/38952.

The bag wherein the front wall is provided with an opening provided with cover, is an outer-bag in a two-bag system. In use, the user or nurse will apply the ostomy bag (the outer bag) as described. Then, though the opening in the front wall an inner-bag is placed inside the outer-bag, and the hole in the inner-bag attached to the adhesive wafer. When the inner-bag is in place, the hole in the front wall is closed. For replacing the inner-bag, the hole in the front wall is reopened, and the hole in the inner-bag attached to the adhesive wafer is detached from the adhesive wafer. Then the inner-bag is withdrawn from the outer-bag. A new inner-bag can now be placed in the clean outer-bag as described above.

The adhesive wafer may comprise any skin-friendly adhesive known per se, e.g. an adhesive comprising hydrocolloids or other moisture absorbing constituents for prolonging the time of use forming the surface to be secured to the user's skin. The adhesive may e.g. be of the type disclosed in those disclosed in US patent Nos. 4,367,732, 5,051.259, 5,714,225, 6,171,594, 6.303,700, 6,451,883, or 6,437,038, or in WO Publication Nos. 00/54820, or 01/05340.

A sealing member may suitably be made of a material of the kind disclosed in US Patent No. 6,509,391, or 6,332,879. Thus, the elastic and plastic properties may be selected by the skilled in the art in accordance with the desired properties for the actual purpose, including a self-sealing effect obtainable using a sealing member made from an adhesive material showing absorbing and swelling properties when contacted by aqueous material.

A carrier sheet may be present on the surface of the adhesive wafer facing the bag and may any suitable thermoplastic material known per se for use in the preparation of ostomy appliances e.g. a foam, a non-woven layer or a polyurethane, polyethylene, polyester or polyamide suitable for attachment of a collecting bag using an adhesive or by welding.

The collection bag may be made in analogy with and from materials conventionally used for the preparation of ostomy appliances. Such materials are suitably films composed of any suitable material, which is heat sealable and sufficiently impervious for unpleasant odours such as polyolefin films or combinations of such films, e.g. polyethylene or a coextrudate of polyethylene and polyvinylidene chloride. Suitably the bag is made from front and rear walls welded in a manner known per se along the rim forming a bag. When cutting or punching the walls, an opening for receiving a stoma is suitably also punched in the wall to form the rear wall.

A protective cover or release liner may for instance be siliconized paper. It does not need to have the same contour as the dressing, e.g. a number of dressings may be attached to a larger sheet of protective cover. The protective cover is not present during the use of the dressing of the invention and is therefore not an essential part of the invention.

### Description of the Preferred Embodiments

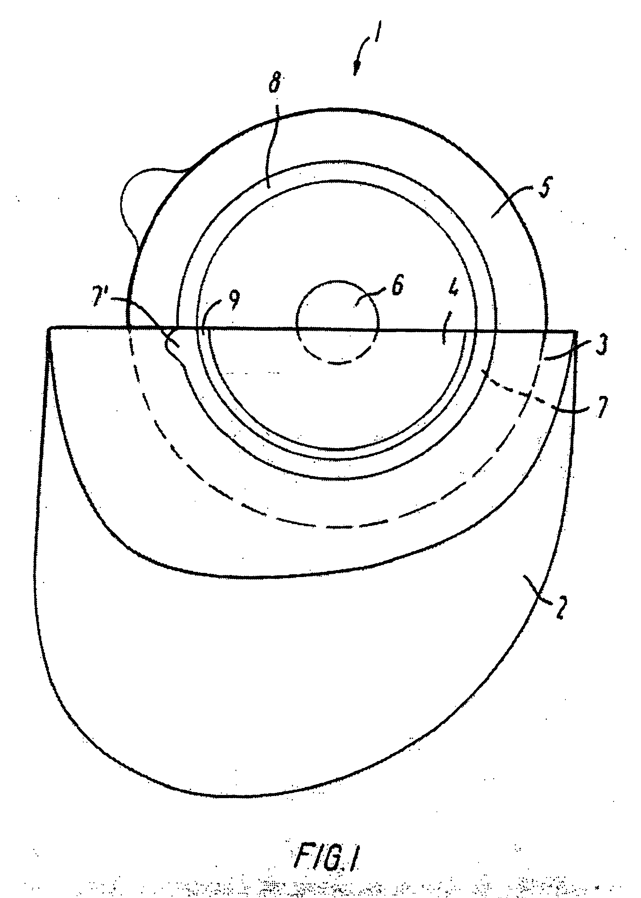

The invention is now explained more in detail with reference to the drawings showing examples of an ostomy appliance.
Reference is made to figure 1 showing an ostomy appliance **1** in its open state. The ostomy appliance comprises a front wall **2** and a rear wall 3 of flexible material forming a bag, said rear wall having an opening **4** for receiving a stoma, and said rear wall also being provided with an adhesive wafer **5** for securing the appliance to a user's skin, said wafer having a hole **6** being aligned with the opening for receiving the stoma, wherein the opening has an edge **7,7'** being secured to the surface of the adhesive wafer facing away from the user in an attachment zone **8** surrounding the hole of the wafer, wherein a first part of the edge **7** of the bag is secured to the surface of the wafer in a corresponding first part of the attachment zone, and wherein a second, remaining part **7'** of the edge of the bag is prepared for adhesive sealing of the same to the remaining second part **8** of the attachment zone. The edge of the opening of the bag is provided with a stiffening element **9**.

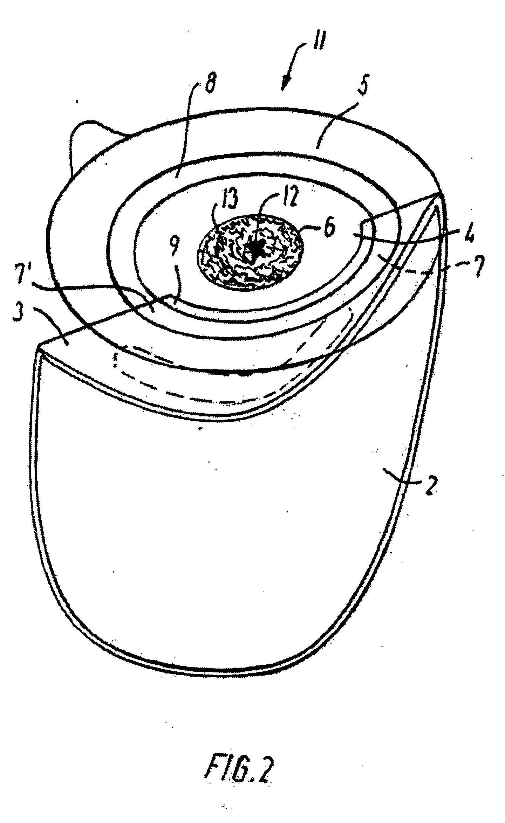

Fig. 2 shows an ostomy appliance **11** in its open state applied to a users abdomen. The ostomy appliance comprises a front wall **2** and a rear wall **3** of flexible material forming a bag, said rear wall having an opening **4** for receiving a stoma **12,** and said rear wall also being provided with an adhesive wafer **5** for securing the appliance to a user's skin, said wafer having a hole **6** being aligned with the opening for receiving the stoma, wherein the opening has an edge **7,7'** being secured to the surface of the adhesive wafer facing away from the user in an attachment zone **8** surrounding the hole of the wafer, wherein a first part of the edge **7** of the bag is secured to the surface of the wafer in a corresponding first part of the attachment zone, and wherein a second, remaining part **7'** of the edge of the bag is prepared for adhesive sealing of the same to the remaining second part **8** of the attachments zone. The edge of the opening of the bag is provided with a stiffening element **9.** In the hole **6** of the wafer is a separate sealing member **13** sealing against the stoma and the edge of the hole of the wafer.

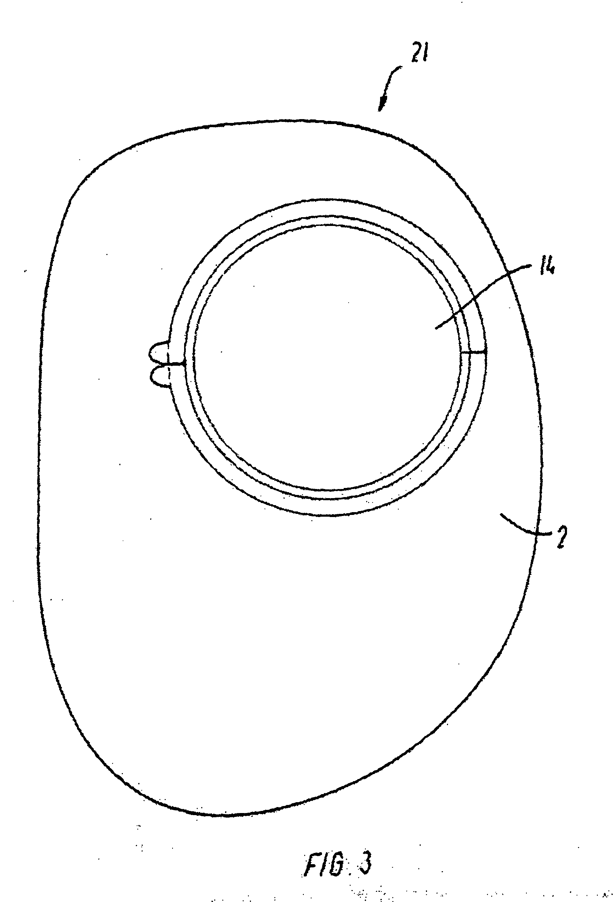

Fig. 3 shows an ostomy appliance **21** in its dosed state seen from the surface facing away from the user. The ostomy appliance corresponds to the embodiment shown in Fig.1 except that in front wall **2** is provided with an opening provided with cover **14,** which may be released and resealed and thus giving temporary access to the internal space of the bag for inspection of the stoma area during the service time of the ostomy appliance.

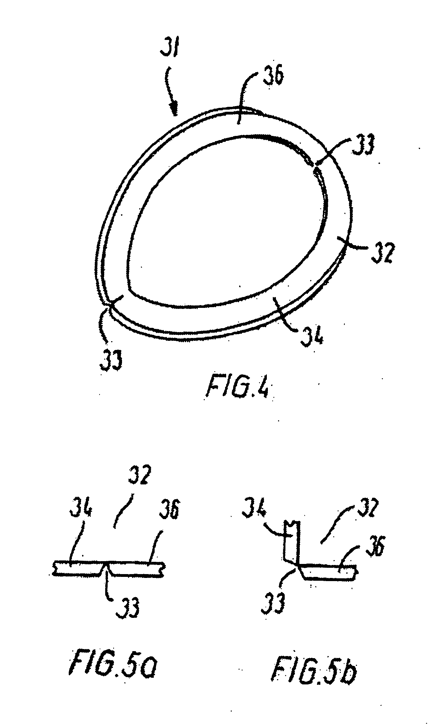

Figs. 4, 5a and 5b show a stiffening element **31** for use in an ostomy appliance of the invention. The stiffening element is in the form of a circular ring **32** provided with two notches **33** dividing the circular ring into two parts - a first part **34** and a second part **36.** The notches provide predetermined zones in which the stiffening element is readily folded. The notches are in this embodiment placed on the surface to be attached to an adhesive wafer of an ostomy appliance of the invention.

## Claims

1. An ostomy appliance (1,21,31) comprising a front wall (2) and a rear wall (3) of flexible material forming a bag, said rear wall (3)
- having an opening (4) for receiving a stoma (12), and
- being provided with an adhesive wafer (5) for securing the appliance (1) to a user's skin, said wafer (5) having a hole (6) being aligned with the opening (4) for receiving the stoma (12)
wherein the opening (4) of the rear wall (3) has an edge (7,7') being adapted to be secured to a surface of the adhesive wafer (5) facing away from the user in an attachment zone (8) surrounding the hole (4) of the wafer (5), and
**characterized in that**
- a first part (7) of the edge (7,7') of the opening (4) of the rear wall (3) is permanently secured to the surface of the wafer (5) in a corresponding first part of the attachment zone (8), and
- a second, remaining part (7) of the edge (7,7') of the opening (4) of the bag is prepared for adhesive reversible coupling of the same to a remaining second part of the attachment zone (8)
wherein the ostomy appliance further comprises an inner bag.

2. An ostomy appliance (1,21,31) as claimed in any of the preceding claims, wherein the second part (7') of the edge (7,7') of the bag is secured to one surface of a stiffening element (9).

3. An ostomy appliance (1 ,21 ,31) as claimed in claim 2, wherein the first (7) and the second (7') part of the edge (7,7') of the bag are secured to a first and a second part, respectively, of one surface of the stiffening element (9).

4. An ostomy appliance (1,21,31) according to any of the preceding claims, wherein the stiffening element (9) is a flat ring (32) provided with two notches (33) dividing the flat ring (33) into two parts (34,36).

5. An ostomy appliance (1,21,31) as claimed in any of the preceding claims, wherein the notches (33) stretch essentially along a chord of the flat ring (32).

6. An ostomy appliance (1,21,31) as claimed in any of the preceding claims, wherein the notches (33) stretch essentially along a diameter of the flat ring (32).

7. An ostomy appliance (1,21,31) as claimed in any of the preceding claims, wherein the part of the stiffening element (9) secured the second part of the bag is provided with an adhesive layer being covered by a protecting layer.

8. An ostomy appliance (1 ,21 ,31) as claimed in any of the preceding claims, wherein the second part of the attachment zone (8) of the wafer (5) is provided with an adhesive layer being covered by a protecting layer.

## Patentansprüche

1. Stomavorrichtung (1, 21, 3 1), die eine vordere Wand (2) und eine hintere Wand (3) aus einem flexiblen Material aufweist, die einen Beutel bilden, wobei die hintere Wand (3)
- eine Öffnung (4) zur Aufnahme eines Stomas (12) aufweist und
- mit einem Klebeplättchen (5) zur Befestigung der Vorrichtung (1) auf der Haut eines Anwenders versehen ist, das ein Loch (6) aufweist, das zur Öffnung (4) zur Aufnahme des Stomas (12) ausgerichtet ist,
wobei die Öffnung (4) der hinteren Wand (3) einen Rand (7, 7') aufweist, der so ausgebildet ist, dass er an einer Oberfläche des Klebeplättchens (5), die vom Anwender abliegt, in einer Anbringungszone (8), die das Loch (6) des Klebeplättchens (5) umgibt, befestigt werden kann,
**dadurch gekennzeichnet, dass**
- ein erster Teil (7) des Randes (7, 7') der Öffnung (4) der hinteren Wand (3) dauerhaft an der Oberfläche des Klebeplättchens (5) in einem entsprechenden ersten Teil der Anbringungszone (8) befestigt ist und
- ein zweiter, verbleibender Teil (7') des Randes (7, 7') der Öffnung (4) des Beutels für eine reversible Befestigung an einem verbleibenden zweiten Teil der Anbringungszone (8) vorbereitet ist,
wobei die Stomavorrichtung ferner einen Innenbeutel aufweist.

2. Stomavorrichtung (1, 21, 31) nach dem vorhergehenden Anspruch, bei welcher der zweite Teil (7') des Randes (7, 7') des Beutels an einer Oberfläche eines versteifenden Elements (9) befestigt ist.

3. Stomavorrichtung (1, 21, 31) nach Anspruch 2, bei welcher der erste Teil (7) und der zweite Teil (7') des Randes (7, 7') des Beutels an einem ersten bzw. einem zweiten Teil einer Oberfläche des versteifenden Elements (9) befestigt sind.

4. Stomavorrichtung (1, 21, 31) nach einem oder mehreren der vorhergehenden Ansprüche, bei der das versteifende Element (9) ein flacher Ring (32) ist, der mit zwei Einschnitten (33) versehen ist, die den flachen Ring (32) in zwei Teile (34, 36) unterteilen.

5. Stomavorrichtung (1, 21, 31) nach einem oder mehreren der vorhergehenden Ansprüche, bei der sich die Einschnitte (33) im Wesentlichen längs einer Sehne des flachen Rings (32) erstrecken.

6. Stomavorrichtung (1, 21, 31) nach einem oder mehreren der vorhergehenden Ansprüche, bei der sich die Einschnitte (33) im Wesentlichen längs eines Durchmessers des flachen Rings (32) erstrecken.

7. Stomavorrichtung (1, 21, 31) nach einem oder mehreren der vorhergehenden Ansprüche, bei welcher der Teil des versteifenden Elements (9), der am zweiten Teil des Beutels befestigt ist, mit einer Klebeschicht versehen ist, die mit einer Schutzschicht abgedeckt ist.

8. Stomavorrichtung (1, 21, 31) nach einem oder mehreren der vorhergehenden Ansprüche, bei welcher der zweite Teil der Anbringungszone (8) des Klebeplättchens (5) mit einer Klebeschicht versehen ist, die mit einer Schutzschicht abgedeckt ist.

## Revendications

1. Dispositif pour stomie (1, 21, 31) comprenant une paroi avant (2) et une paroi arrière (3) constituées en une matière souple formant une poche, ladite paroi arrière (3)
- possédant un orifice (4) destiné à recevoir une stomie (12) et
- étant munie d'une bande adhésive (5) destinée à fixer le dispositif (1) sur la peau d'un utilisateur, ladite bande (5) possédant un trou (6) qui est aligné avec l'orifice (4) destiné à recevoir la stomie (22)
dans lequel l'orifice (4) de la paroi arrière (3) a un bord (7, 7') qui est adapté pour être fixé à une surface de la bande adhésive (5) disposée au contact de la peau de l'utilisateur dans une zone de fixation (8) entourant le trou (6) de la bande (5), et
**caractérisé en ce que**
- une première partie (7) du bord (7, 7') de l'orifice (4) de la paroi arrière (3) est fixée de manière permanente à la surface de la bande (5) dans une première partie correspondante de la zone de fixation (8), et
- une deuxième partie (7') restante du bord (7, 7') de l'orifice (4) de la poche est préparée pour l'accouplement adhésif réversible de celui-ci à une deuxième partie restante de la zone de fixation (8)
le dispositif pour stomie comprenant en outre une poche intérieure.

2. Dispositif pour stomie (1, 21, 31) comme revendiqué dans la revendication précédente, dans lequel la deuxième partie (7') du bord (7, 7') de la poche est fixée à une surface d'un élément rigidifiant (9).

3. Dispositif pour stomie (1, 21, 31) comme revendiqué dans la revendication 2, dans lequel la première (7) et la deuxième (7') partie du bord (7, 7') de la poche sont fixées à une première et une deuxième partie, respectivement, d'une surface de l'élément qui lui donne sa rigidité (9).

4. Dispositif pour stomie (1, 21, 31) comme revendiqué dans l'une quelconque des revendications précédentes, dans lequel l'élément qui lui donne sa rigidité (9) est un anneau plat (32) muni de deux encoches (33) divisant l'anneau plat (32) en deux parties (34, 36).

5. Dispositif pour stomie (1, 21, 31) selon l'une quelconque des revendications précédentes, dans lequel les encoches (33) s'étirent sensiblement le long d'une corde de l'anneau plat (32).

6. Dispositif pour stomie (1, 21, 31) comme revendiqué dans l'une quelconque des revendications précédentes, dans lequel les encoches (33) s'étirent sensiblement le long d'un diamètre de l'anneau plat (32).

7. Dispositif pour stomie (1, 21, 31) comme revendiqué dans l'une quelconque des revendications précédentes, dans lequel la partie de l'élément qui lui donne sa rigidité (9) fixée à la deuxième partie de la poche est munie d'une bande adhésive qui est recouverte d'une couche protectrice.

8. Dispositif pour stomie (1, 21, 31) comme revendiqué dans l'une quelconque des revendications précédentes, dans lequel la deuxième partie de la zone de fixation (8) de la bande (5) est munie d'une couche adhésive qui est recouverte d'une couche protectrice.
